Europäisches Patentamt

European Patent Office

Office européen des brevets

(19)

(11) Publication number: **0 180 543 B1**

# (12) EUROPEAN PATENT SPECIFICATION

(45) Date of publication of patent specification: **24.04.91**  (51) Int. Cl.⁵: **A61J 3/07**

(21) Application number: **85810478.9**

(22) Date of filing: **18.10.85**

(54) Method for sealing capsules and capsule.

(30) Priority: **25.10.84 CH 5101/84**
**21.03.85 GB 8507384**

(43) Date of publication of application:
**07.05.86 Bulletin 86/19**

(45) Publication of the grant of the patent:
**24.04.91 Bulletin 91/17**

(84) Designated Contracting States:
**AT BE CH DE FR GB IT LI LU NL SE**

(56) References cited:
**EP-A- 0 116 743**      **EP-A- 0 116 744**
**EP-A- 0 152 517**      **DE-A- 2 232 236**
**DE-A- 2 729 007**      **FR-A- 1 574 462**
**FR-A- 2 204 541**      **FR-A- 2 235 848**
**US-A- 4 026 986**      **US-A- 4 040 536**
**US-A- 4 281 763**      **US-A- 4 522 666**

(73) Proprietor: **WARNER-LAMBERT COMPANY**
**201 Tabor Road**
**Morris Plains New Jersey 07950(US)**

(72) Inventor: **Bodenmann, Hans-Ulrich**
**Tannenstrasse 16**
**CH-4142 Münchenstein(CH)**
Inventor: **Wittwer, Fritz**
**Im Budler 15**
**CH-4419 Lupsingen(CH)**
Inventor: **Cadé, Dominique**
**25, Rue Saint Joseph**
**F-68000 Colmar(FR)**
Inventor: **Mayer, Jean Philippe**
**59, Rue des 3 Chateaux**
**F-68000 Colmar(FR)**

(74) Representative: **Silbiger, Jakob, Dr.**
**c/o CAPSUGEL AG Fabrikmattenweg 2-4**
**CH-4144 Arlesheim(CH)**

## Description

The present invention refers to a method for sealing capsules using specially designed hard shell capsules, and to said hard shell capsules.

Hard shell capsules are generally made from gelatin or other hydrophylic materials, preferably from gelatin using the dip-moulding technique. These capsules have cylindrical, telescopically joinable coaxial cap and body parts, each having a side wall, an open end and a closed end, the cap and the body being adapted to be mutually joined. These capsules generally have some locking means. A typical hard shell gelatin capsule for example is being locked by a matching contact of a circumferential body groove with ridge means extending inward from the inner cap wall.

These capsules are preferably used as a container resp. for the exact dosage for substances, e.g. pharmaceuticals and are preferably made from a pharmaceutically acceptable grade of gelatin or other substances similar to gelatin in physical and chemical properties.

These capsules have the disadvantage that the cap and body parts can be separated and rejoined without the opening of the capsule becoming externally visible or tamper-evident.

It has therefore been suggested to seal these capsules with a sealing fluid, preferably a mixture of alcohol and water by evenly distributing the sealing fluid within the overlap of the cap and body part of the capsule and leaving this wetted part drying at room temperature or applying heat to it. Such sealing processes are described in the European Patent Applications No. 83 305 331.7 (Publ. No. 116 744) and No. 33 305 330.9 (Publ. No. 116 743). These methods described hitherto give tamper-proof sealing, especially when heat is applied for the sealing process. Under the same conditions of application of heat it is possible to obtain liquid-proof sealings, but so far the nubmer of leaking capsules is too high, especially when the capsules are filled with liquids of a low viscosity. The requirements as to the liquid-proofness and the technique to achieve it are very high taking the increasing speed of the filling and controlling machines into account and that the speed of the sealing process must equal that of the filling machine.

FR-A-2-204 541 discloses two piece hard shell locking capsules. However, the cap and body of the capsules disclosed by FR-A-2-204 541 are not necessarily maintained in an exactly coaxial relationship by the claimed closing arrangement. Such prior capsules are prone to leak liquid contents.

It has now surprisingly been found that using a certain new type of capsule gives excellent results on sealing with respect to quality and speed of sealing; the number of leaking capsules being negligible.

The present invention relates in particular to a method for sealing hard gelatin capsules having coaxial cap and body parts which overlap when telescopically joined, by contacting the edge of the cap part of the capsule with the sealing liquid so that it gets evenly distributed within this overlapping section, (ii) removing the excess of the sealing liquid from the exposed outside surface so that only the overlapping section remains wet and (iii) causing the sealing of the capsule within the overlapping section of the capsule body and cap parts by leaving the capsule at ambient temperature or by applying heat to said overlapping section, characterized in that a capsule is used wherein the cap part has on its inner surface wall an annular ridge or an arrangement which functions as an annular ridge said arrangement being preferably an arrangement of ridge segments and/or protrusions and spaced from said ridge or said arrangement towards the open end of the cap part there is arranged means at a spacing being sufficient to hold cap and body in an exactly coaxial position.

The invention also includes a hard gelatin capsule having cylindrical telescopically joinable coaxial cap and body parts each having a side wall, an open end and a closed end, the cap and the body being adapted to be mutually joined, wherein the capsule is one wherein the cap part has on its inner surface wall an annular ridge or an arrangement which functions as an annular ridge, characterized in that the ridge(s) or arrangement(s) is-(are) located at the upper part of the cap near its closed end within the upper 50 % of the cap length calculated to the total length of the cap, and that spaced from said ridge or said arrangement towards the open end of the cap part, there is arranged means comprising at least three protrusions at a spacing sufficient to hold the cap and body in an exactly coaxial position, said means being located within 50 to 95 % of the total cap length calculated from the top of the closed end of the cap towards its open end.

The closed ends of the cap and body parts may be hemispherical, conical, pyramidical, flat or may have any other form. Preferably they are hemispherical, especially for dip-molded capsules.

The annular ridge of the cap part is preferably circumferential and may be interrupted. The arrangement which functions as a ridge may e.g. be a number of protrusions or ridge segments which are arranged circumferentially. Such annular ridges or arrangements are known. If the cap is long enough there may be an additional annular ridge or arrangement which functions as a ridge.

The cross-section of the ridge can be a ring form or may have the form of a triangle or a polygon. The form, however, is not critical and depends on the manufacturing process of the cap part.

The means to hold the cap and body in an exactly coaxial position does not follow directly the annular ridge described above but is spaced at a spacing long enough so as to properly exhibit its function. These means can be an annular ridge, preferably an arrangement of ridge segments and/or protrusions so as to hold cap and body in an exactly coaxial position.

The optimum arrangement has shown to be a number of protrusions arranged in an annular ring form, preferably in a symmetrical form, preferably adjacent protrusions having all the same distance from each other. The minimum preferred number are three protrusions.

The depth of the protrusions is so dimensioned that the open end of the cap part and the open end of the body part can be easily joined together; the protrusions contacting the outer side wall of the body part and generating a slight pressure at the point of contact. There are arranged preferably 4, 5, 6, 7, 8, 9 or 10 protrusions, more preferably 6, 7, 8, 9 or 10 and most preferably 6 or 8. hey may have different forms as to the cross-section, diameter, depth etc. Such forms of protrusions are known. Preferably they have all the same form and especially the same depth. It is also possible to use an annular ridge which is preferably interrupted.

As mentioned the distance between the annular ridge, which is situated preferably at the upper part of the cap near its closed end and the means to hold cap and body in an exactly coaxial position is important. The means to hold cap and body in the coaxial position should not follow directly the ridge near the end of the cap. Of course the actual length of this distance depends on the size of the capsule resp. the cap.

The annular ridge or the arrangement which functions as a ridge is located preferably at the upper part of the cap near its closed end, preferably within the upper 50% of the cap length, calculated to the total length of the cap and preferably within the upper 33% - 45% of the cap length near the closed end, calculated to the total length of the cap.

The means to hold cap and body in an exactly coaxial position is located preferably within 50% to 95% of the cap length, calculated from the top of the cap, towards its open end, preferably within 50% to 85% of the total cap length calculated from the top of the closed end of the cap towards its open end, preferably within 55 - 80% and preferably within 65-75% of the total cap length calculated from the top of the closed end of the cap towards its open end. Within the range of 65 - 75% also two protrusions lead to acceptable results, especially for longer size caps, i.e. longer than 7 mm, pref. longer than 8 mm.

The distance between the upper ridge and the means to hold cap and body in an exactly coaxial position is preferably not less than 2 mm preferably not less than 2,5 mm. These measures are independant of the capsule size and especially suitable for the known capsule sizes 000, 00, 0, 1, 2, 3, 4, 5. For larger lengths of the cap the mentioned distances between ridge and the means may be also longer, preferably 3-5 mm, and for long caps also more than 5 mm depending on the length of the cap.

It is also possible to have more than one such means to hold cap and body in an exactly coaxial position especially if the cap is long enough, for example if the cap encloses the greater part of the cylindrical body side wall or its whole length. Two ridges and/or two means may follow each other and are preferably situated within the limits given above.

The body part may be smooth, i.e. without ridges or grooves. Preferably the body part has on its outer surface annular grooves or an arrangement of grooves matching with the ridge means of the inner surface of the cap so as to provide a substantially distortion-free, full lock between the cap and the body.

If the capsule is pre-locked the protrusions match preferably with the groove means of the body part. The dimensions can easily be chosen by a person skilled in the art.

The annular ridges and grooves may be interrupted in such a way that the spaces between the ridge segments act as vents to permit air to escape from within the capsule when joined.

The annular ridge of the cap may be represented by a constriction of the diameter of the cap or may have two slopes with an optional flat surface in between. The angles of the slopes or of the constriction are not critical and limited only by the limitation given by the manufacturing process. It means for instance that angles which cause an entrapment of air in the commonly used dipping process are to be avoided, which is known to the man skilled in the art.

The same is to be said for the dimensions of the protrusions. Different types of protrusions have become known and they are all suitable. Their basis can be e.g. oval, round or rectangular. The cross-section may have a round form or the form of a triangle or a polygon, e.g. two slopes and an optional flat surface in between. The angles of the slopes are not critical and only limited by the limitations given by the manufacturing process.

A further embodiment of the present invention is that the body has a reduced diameter of the outer wall in the area of its open end compared to the diameter of the rest of the outer wall. This reduces the danger of an abutment of the free edges of the capsule body and the capsule cap when they are telescoped. The dimension of the constriction is not critical. Preferably the axial width of the recess is about 10 to 20 times as large as its depth if the capsule body is produced by the dip-molding process. Preferably the contriction of the body matches with the closed end of the cap or a constriction of the cap to give a tight mechanical seal when the capsule is closed.

A further embodiment of the present invention is that the body has no constriction at the end but its length is set in such a way so as to match with the closed end of the cap or a constriction of the cap to give a tight mechanical seal when the capsule is closed.

A further embodiment of the invention is characterized in that the body closed end has a hemispheroidal, conical, pyramidal or flat outside surface and that the cylindrical body side wall is totally enclosed within the inner cap side wall when the capsule is joined. Preferably the cylindrical outer side wall of the body covers practically completely the inner cylindrical side wall of the cap.

Capsules according to the present invention can be prepared by the dip molding process from gelatin in a manner known per se. It is also possible to prepare them from hydrophilic materials like gelatin or starch derivatives or mixtures thereof or from native starch by injection molding as described in the European Patent Applications Nos. 83 301 642.1 and 84 300 940.8. Capsules according to the present invention and as described herein are new and are part of the present invention. They are preferably made from gelatin by the dip molding process or from gelatin or starch by injection molding, preferably from gelatin by the dip molding process.

The sealing process is known per se. The invention resides as mentioned in the use of the above described capsules within this process. It was surprisingly found that the capsules of the present invention are extremely well suited for these liquid sealing processes. Cap and body being in an exactly coaxial position means that the two rings that appear in the horizontal cross-section of the overlapping section of the cap and the body have at any height a same and common central point whereby deviation from the round form/or ovalisation is reduced to a minimum. It is a surprising perception that known capsules do not fulfill this condition but leave open a free movement between cap and body parts even they are coaxially joined, thus preventing an exactly coaxial position. This is shown in Fig. A. The coaxial position appears to be essential for the present sealing process.

The sealing process according to the present invention is essentially carried out (i) by contacting the edge of the cap part of the capsule with the sealing liquid so that it gets evenly distributed within this overlapping section, mainly by capillary forces, (ii) removing the excess of the sealing liquid from the exposed outside surface so that only the overlapping section remains wet and (iii) causing the sealing of the capsule within the overlapping section of the capsule body and cap parts by leaving the capsule at ambient temperature or by applying heat to said overlapping section.

Contacting the edge of the cap part of the capsule with the sealing liquid can be carried out by any suitable means, e.g. dipping within the fluid, spraying, contacting a solid material impregnated or wetted with the sealing fluid. Dipping or spraying, especially spraying is preferred.

The excess sealing fluid is removed by any suitable means such as agitating, vibrating, blowing off with air or a combination thereof. Preferred is the blowing off with air at ambient temperature, e.g. in a fluidized bed.

Causing the sealing of the capsule can be achieved simply by leaving the capsule drying at ambient temperature. However, the preferred sealing is carried out at elevated temperature.

Such elevated temperature is higher then $30^\circ$ C preferably 30 - 80$^\circ$ C, preferably 35 - 70$^\circ$ C. Most preferred is a temperature of 40 - 60$^\circ$ C. The temperature may be reached by using convection energy e.g. by heating with heated air of the appropriate temperature, i.e. also higher than $30^\circ$ C, resp. of 30 - 80$^\circ$ C, preferably 35 - 70$^\circ$ C and preferably 40 - 60$^\circ$ C, e.g. using a fluidized bed or any other arrangement which prevents the capsules from sticking together and provides an even distribution of the temperature within the overlapping section of the cap and body part of the capsule.

The temperature and temperature limits of the air are not critical. Air of a higher temperature can be used in combination with shorter heating times. Heating can also be carried out using electro magnetic energy such as infra-red waves. By this method it is preferred to create the above mentioned temperature within the overlapping section of the capsule containing the sealing fluid.

Water and many organic liquids have been described as being suitable sealing fluids. Preferred is a water soluble organic fluids containing water. Most preferred are aliphatic monohydric alcohols having one to four carbon atoms which may be substituted by one alkoxy group having one or two carbon atoms or mixture thereof containing 2 - 90% of water, preferably 5 - 70% of water. " % " are percent by volume.

Preferred monohydric alcohols are n-propanol, 2-propanol, ethanol and methanol or mixtures thereof, most preferred is ethanol resp. a ethanol/water mixture. The alcohol content is preferably more than 40%, preferably 40 - 95%, most preferred is 45 - 93%.

To reduce the surface tension of the mixture resp. to facilitate its even distribution within the overlap of the cap and body parts a surface active agent may be added.

The optimum sealing temperature largely depends on the ratio of the water soluble liquid to the water. Taken an ethanol/water mixture it may be said that the higher the water content is, the lower the sealing temperature can be chosen. An ethanol content preferably of below 70%, preferably below 55% is used at ambient sealing temperature up to 30°C. However, for an ethanol content of 30 - 85% a sealing temperature of 30 - 60°C and for a ethanol content of 65 - 95% a sealing temperature of 40 - 70°C will generally be appropriate.

The higher the temperature the shorter will be the sealing time. The mentioned limits are not critical and to be taken as indication for preferred values. It is no problem for the expert to find the optimum value of temperature and time for each individual case. These limits are especially suitable for ethanol/water mixtures.

The method of the present invention is particularly suitable to seal capsules containing liquids, like organic or mineral oils or liquid pharmaceutical preparation although solid or pasty masses may be filled into the capsules. Liquid oils and preparations of low viscosity may counteract the sealing process by leaking out into the overlapping section of cap and body part. It is therefore surprising that such excellent results are obtained.

Fig. A shows the non-coaxial arrangement of a conventional capsule.

Fig. 1a, 1b and 1c show a side elevation view of preferred capsule shapes.

Fig. 2 is a side-sectional view (along the 2-2-axis of Fig. 1a) of the locking section of a preferred embodiment in completely locked form.

Fig. 3 corresponds to a capsule according to Fig. 2, but in pre-locked form.

Fig. 4 is a side-sectional view of the principle of a body part with a diameter restriction at the open end.

Fig. 5 shows a side elevation view of an embodiment of the present invention wherein the cap covers completely the cylindrical wall of the body, the body having a spherical end.

Fig. 6 is a side-sectional view of Fig 5.

Fig. 7 shows a side elevation view of an embodiment of the present invention differing from Fig. 5 resp. Fig. 6 by having two annular ring systems.

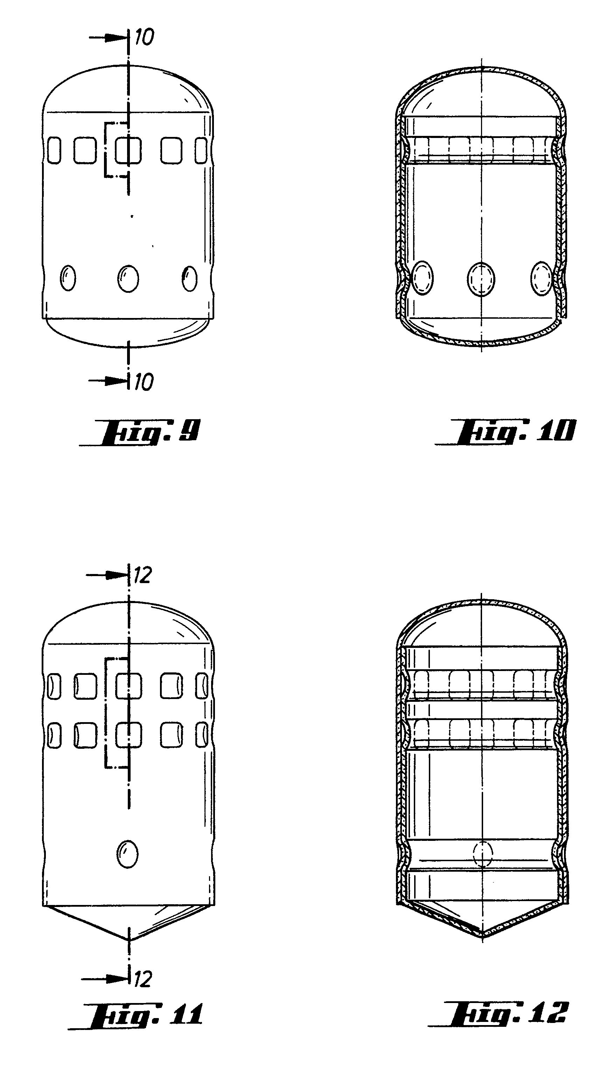

Fig. 8 is a side-sectional view of Fig. 7.

Fig. 9 shows a side elevation of a further embodiment, analogous to Fig. 5, having. air vents, 6 protrusions and a "flat" end of the body.

Fig. 10 shows a side-sectional view of Fig. 9.

Fig. 11 shows a side elevation view of a further embodiment of the present invention. Fig. 12 shows the side-sectional view of Fig. 11.

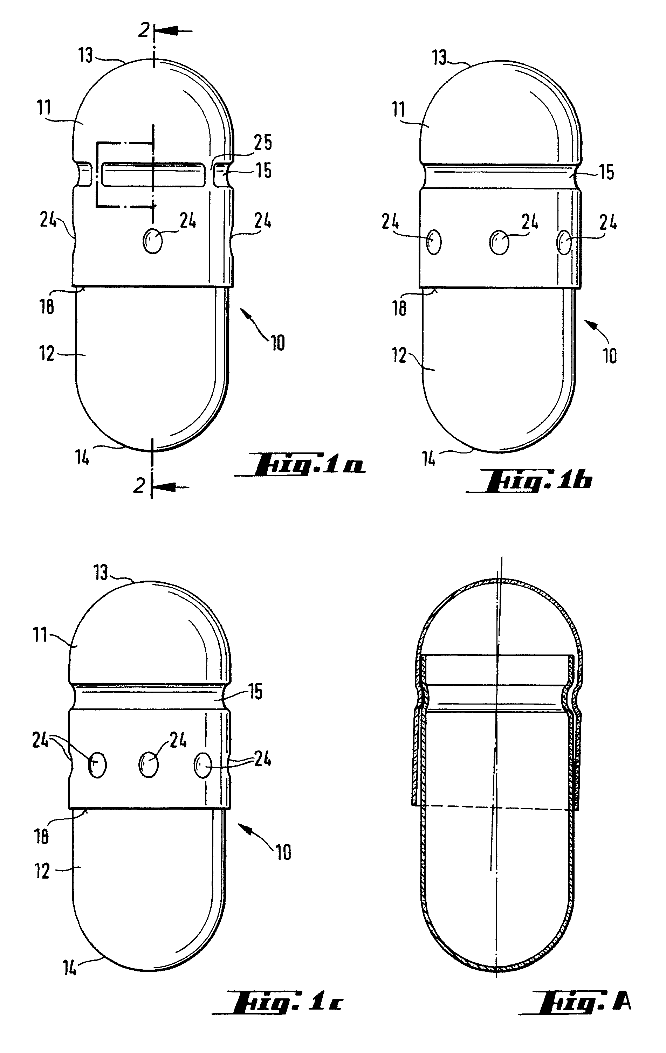

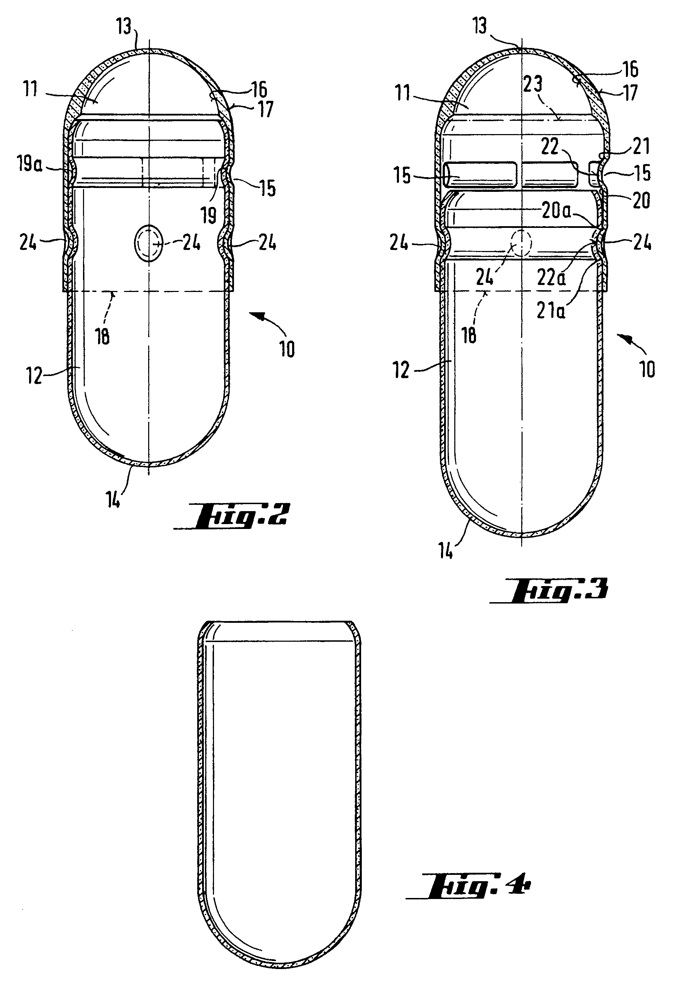

The capsules 10 shown in Fig. 1a), 1b) and 1c) have a cap part 11 and a body part 12, both being closed at the ends 13 resp. 14. The cap 11 has a circumferential ridge 15, which may be interrupted by air vents. The cap has an open end and between this open end and the ridge 15, there are four (Fig. 1a), six (Fig. 1b) or eight (Fig. 1c) protrusions 24. The protrusions 24 need not necessarily all have the same form. The details of the cap 11 with the outer wall 17 and the inner wall 16 are shown in Fig. 2 and Fig. 3. The inner wall 16 of the cap shows a ridge 19 corresponding to the restriction 15 shown on the outer wall. The ridge 19 has an angular cross-section, shown on the inner wall with the bevels 20 and 21 meeting at the apex 22.

The closed end 13 is preferably rounded or hemispherical but the shape is not critical. If desired, the cap end can have other shapes. The inner cap wall 16 proceeding from the open end 18 to the line 23, which is the shoulder line, has a slight narrowing diametral taper of the order of 0.01 cm per cm exclusive of ridge 19 and indent means 24.

In Figure 2 the cap and body parts are shown in the fully locked position whereas in Figure 3 the cap and body parts are shown in partly closed or pre-locked position with the open end of the body advanced towards the leading bevel 20 of the ridge 19. The body has a groove 19a which matches the ridge 19. Groove 19a has a leading bevel 20a and a trailing bevel 21a which join at apex 22a. In Figure 2, as indicated, the cap and body have been pressed together from the partly closed pre-locked or semi-locked position into the fully closed, locked position. Here the contriction at the end of the body matches with the cap to give a tight mechanical seal. In the locked position ridge 19 and body groove 19a are in a matching fit or mechanical fit as distinguished from a friction fit, with their respective bevels and apexes in close conformity. In this position the open body end has advanced into the cap to a point near or preferably just beyond the shoulder line 23. The body like the cap is tapered in the same degree and in the direction from its open end to its closed end. The body taper and the body dimensions in relation to the taper and dimensions of the cap also are such as to provide a relatively distortion-free fit in the pre-locked position shown in Figure 3; the fit between adjacent wall surfaces of the cap and body advantageously permits the passage of air. The pre-locked fit in the area of the indent 24 is preferably a mechanical fit as distinguished from a friction fit so that it is substantially distortion-free.

This construction provides for increased passage means or air vent means 25 so as to permit the escape of compressed air contained within the capsule occasioned, for example, by the sudden joining of the body and cap parts into locked position. Thus, the release of air advantageously avoids any tendency of the cap and body to reopen after filling.

Capsules according to the present invention can be used as containers resp. for the exact dosage for food stuffs pharmaceuticals, chemicals, dyestuffs, spices, fertilizing combinations, seeds, cosmetics and agricultural products and matrices of various shapes and sizes for food-stuffs, pharmaceuticals, chemicals, dyestuffs, spices, fertilizing combinations, seeds, cosmetics and agricultural products in any useful form such as powder or liquids. Special forms such as microdispersions within the matrix and released from it through disintegration and/or dissolution and/or bioerrosion and/or diffusion resulting in a controlled release delivery system for the enclosed substance, and medical and surgery products, formed from the compositions or the foams thereof can also be filled into capsules of the present invention.

Example 1

10,000 gelatin capsules size 2, of the form as shown in Fig. 1b, natural transparent, were automatically filled with each 0.320 g of peanut oil (French Codex grade) and closed, at a rate of 11,000 capsules per hour. These capsules were directly introduced in a random manner into the spraying station where they were contacted with a mixture of 55% of pharmaceutical grade ethanol and 45% of demineralized water (% by volume of total solution), during about 1 second. The excess of the mixture was drained off by means of a compressed air blast at 20°C. Immediately thereafter, the capsules were continuously fed into a fluidized bed dryer where they were first dried further with air at ambient temperature for 3 minutes and then sealed by means of an air flow of a temperature of 46°C during 3 minutes. The capsules were then discharged from the apparatus. The obtained capsules were completely sealed and effectively liquid proof.

Example 2

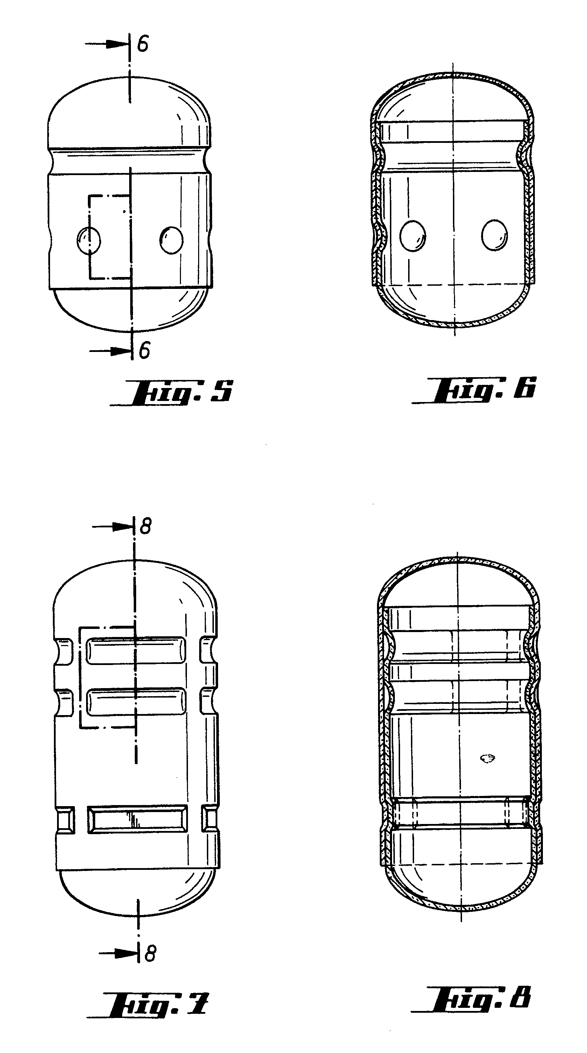

Example 1 was analogously repeated with gelatin capsules of size 3 according to Fig. 5, filled with a vitamin E preparation, using an ethanol/water mixture of 90:10 and a sealing temperature of 60°C during 4 minutes. The capsules were perfectly liquid-proof.

Example 3

Example 1 was analogously repeated using white opaque capsules containing 2% of titanium dioxid by weight. An ethanol/water mixture of 75:25 and a sealing temperature of 55°C during 3 minutes. Excellent liquid-proofness was obtained.

Example 4

Example 2 was analogously repeated using an ethanol/water mixture of 40:60 and a sealing temperature of 30°C during 10 minutes. Excellent liquid proofness was obtained.

Excellent results were also obtained with other sizes filled with solid or pasty masses and other sealing temperatures and sealing times.

## Claims

1. Method for sealing hard gelatin capsules having coaxial cap and body parts which overlap when telescopically joined, by contacting the edge of the cap part of the capsule with the sealing liquid so that it gets evenly distributed within this overlapping section, (ii) removing the excess of the sealing liquid from the exposed outside surface so that only the overlapping section remains wet and (iii) causing the sealing of the capsule within the overlapping section of the capsule body and cap parts by leaving the capsule at ambient temperature or by applying heat to said overlapping section, characterized in that a capsule is used wherein the cap part has on its inner surface wall an annular ridge or an arrangement which functions as an annular ridge said arrangement being preferably an arrangement of ridge segments and/or protrusions and spaced from said ridge or said arrangement towards the open end of the cap part there is arranged means at a spacing being sufficient to hold cap and body in an exactly coaxial position.

2. Method according to claim 1, wherein the annular ridge of the cap part is an circumferential ridge which optionally is interrupted.

3. Method according to claim 1, wherein the annular ridge of the cap part is an arrangement of circumferentially arranged ridge segments or protrusions.

4. Method according to any of the claims 1 - 3, wherein the cap part has an additional annular ridge or an additional arrangement of ridge segments and/or protrusions.

5. Method according to any of the claims 1-4, wherein the ridge(s) is(are) located at the upper part of the cap bar its closed end within the upper 50% of the cap length calculated to the total length of the cap.

6. Method according to any of the claims 1-5, wherein the means to hold cap and body in an exactly coaxial position is represented by a minimum of three protrusions, preferably in a symmetrical arrangement, preferably adjacent protrusions having all the same distance from each other.

7. Method according to claim 6, wherein as said means are arranged 4, 5, 6, 7, 8, 9 or 10 protrusions, preferably 6, 7, 8, 9 or 10, preferably 6 or 8 protrusions, preferably in a symmetrical arrangement, preferably all protrusions having the same form and preferably adjacent protrusions having all the same distance from each other.

8. Method according to any one of claims 1-5, wherein said means is an annular ridge which is preferably interrupted.

9. Method according to any one of the claims 1-8, wherein there are two means to hold cap and body in an exactly coaxial position.

10. Method according to any one of the claims 1-9, wherein the means to hold cap and body in an exactly coaxial position is located within 50 - 95% preferably within 50-85%, preferably within 55-80% and preferably within 65-75% of the total cap length, calculated from the top of the closed end of the cap towards its open end.

11. Method according to claim 10, wherein there are two protrusions within a range of 65 - 75% of the total cap length, calculated from the top of the closed end of the cap towards its open end.

12. Method according to any one of the claims 1-11, wherein the body part has a smooth outside surface.

13. Method according to any one of the claims 1-12, wherein the body part has on its outer surface annular grooves or an arrangement of grooves matching with the ridge means of the inner surface of the cap so as to provide a substantially distortion-free, full lock between the cap and the body.

14. Method according to any one of the claims 1-13, wherein the annular ridges and grooves are interrupted in such a way that the spaces between the ridge segments act as vents to permit air to escape from within the capsule when joined.

15. Method according to any one of the claims 1-14, wherein the annular ridge of the cap is a constriction of the diameter of the cap.

16. Method according to any one of the claims 1-15, wherein the annular ridge has two slopes and an optional flat surface in between.

17. Method according to any one of the claims 1-16, wherein the protrusions have an oval, round or rectangular basis and in the cross-section show two slopes and an optional flat surface in between.

18. Method according to any one of the claims 1-17, wherein the body has a reduced diameter of the outer wall in the area of its open end compared to the diameter of the rest of the outer wall.

19. Method according to claim 18, wherein the constriction of the body matches with the closed end of the cap or a constriction of the cap to give a tight mechanical seal when the capsule is closed.

20. Method according to any one of the claims 1-17, wherein the length Of the body is set to match with the closed end of the cap or a constriction of the cap to give a tight mechanical seal when the capsule is closed.

21. Method according to any one of the claims 1-20, wherein the cylindrical body side wall is totally enclosed within the inner cap side wall when the capsule is joined.

22. Method according to any one of the claims 1-21, sealing gelatin capsules which are made by the dip molding process.

23. Method according to any one of the claims 1-21, sealing capsules made from gelatin or starch derivatives or mixtures thereof or from native starch by injection molding.

24. Method according to any one of the claims 1-22, wherein the wall of the cap and/or the body part represent a foam.

25. Method according to any one of the claims 1-24 sealing capsules containing food stuffs pharmaceuticals, chemicals, dyestuffs, spices, fertilizing combinations, seeds, cosmetics and agricultural products as powder, paste or liquid.

26. Method according to any one of the claims 1-25 containing a liquid.

27. Method according to claim 26, wherein the contacting the edge of the cap part of the capsule with the sealing liquid is by dipping within the fluid, spraying, contacting a solid material impregnated or wetted with the sealing fluid, preferably by dipping or spraying, especially by spraying.

28. Method according to any one of the claims 26 and 27, wherein the excess sealing fluid is removed by agitating, vibrating, blowing off with air or a combination thereof, preferably by blowing off with air at ambient temperature, preferably in a fluidized bed.

29. Method according to any one of the claims 27 and 28, wherein sealing is carried out at ambient temperature.

30. Method according to any one of the claims 27 and 28, wherein sealing is carried out at a temperature of higher than 30°C, preferably at 30 - 80°C, preferably at 35 - 70°C and preferably at 40 - 60°C.

31. Method according to any one of the claims 27 - 29, wherein sealing is carried out in a fluidized bed with heated air.

32. Method according to any one of the claims 27 - 28, wherein sealing is carried out with electro magnetic energy preferably with infra-red waves.

33. Method according to any one of the claims 27 - 32, wherein the sealing fluid is a water soluble organic liquid containing water.

34. Method according to any one of the claims 27 - 33, wherein the sealing fluid is an aliphatic monohydric alcohol having one to four carbon atoms which may be substituted by one alkoxy group having one or two carbon atoms or mixture thereof containing 2 - 90% of water, preferably 5 - 70% of water.

35. Method according to any one of the claims 27 - 34, wherein the sealing liquid is an ethanol/water mixture.

36. Method according to claim 35, wherein the sealing fluid contains more than 40% or ethanol, more preferably 40 - 95%, most preferred 45 - 83%.

37. Capsules sealed according to any one of the claims 1 - 36.

38. A hard gelatin capsule the capsule having cylindrical telescopically joinable coaxial cap and body parts each having a side wall, an open end and a closed end, the cap and the body being adapted to be mutually joined, wherein the capsule is one wherein the cap part has on its inner surface wall an annular ridge or an arrangement which functions as an annular ridge, characterized in that the ridge(s) or arrangement(s) is(are) located at the upper part of the cap near its closed end within the upper 50% of the cap length calculated to the total length of the cap, and that spaced from said ridge or said arrangement towards the open end of the cap part, there is arranged means comprising at least three protrusions at a spacing sufficient to hold the cap and body in an exactly coaxial position, said means being located within 50 to 95% of the total cap length calculated from the top of the closed end of the cap towards its open end.

39. A hard gelatin capsule according to claim 38, wherein spaced towards the open end of the cap part there is arranged means in the form of 4, 5, 6, 7, 8, 9 or 10 protrusions.

**Revendications**

1. Procédé de fermeture de gélules en gélatine dure, comportant des parties de couvercle et de corps coaxiales qui se recouvrant quand elles sont assemblées par emboîtement, qui consiste à mettre en contact le bord de la partie de couvercle de la gélule avec le liquide d'étanchéité de façon qu'il soit uniformément réparti à l'intérieur de cette zone de recouvrement, (ii) à éliminer l'excès du liquide d'étanchéité se trouvant sur la surface extérieure exposée, de façon que seule la zone de recouvrement reste humide, et (iii) à provoquer l'étanchéité de la gélule à l'intérieur de la zone de recouvrement des parties de corps et de couvercle, en laissant la gélule à la température ambiante ou en appliquant de la chaleur à ladite zone de recouvrement, caractérisé en ce qu'on utilise une gélule dont la partie de couvercle comporte, sur sa paroi de surface intérieure, une nervure annulaire ou un agencement jouant le rôle d'une nervure annulaire, cet agencement étant de préférence un agencement constitué de segments de nervure et/ou de saillies, et en ce qu'à distance de ladite nervure ou dudit agencement, vers l'extrémité ouverte de la partie de couvercle et de corps, sont disposés des moyens sous un espacement suffisant pour maintenir le couvercle et le corps dans une position exactement coaxiale.

2. Procédé selon la revendication 1, dans lequel la nervure annulaire de la partie de couvercle est une nervure périphérique facultativement interrompue.

3. Procédé selon la revendication 1, dans lequel la nervure annulaire de la partie de couvercle est un agencement de segments de nervure ou de saillies disposés de manière périphérique.

4. Procédé selon l'une quelconque des revendications 1 à 3, dans lequel la partie de couvercle comporte une nervure annulaire supplémentaire ou un agencement supplémentaire de segments de nervure et/ou de saillies.

5. Procédé selon l'une quelconque des revendications 1 à 4, dans lequel la ou les nervure(s) sont situées à la partie supérieure du couvercle, au voisinage de son extrémité fermée, dans les 50% supérieurs de la longueur du couvercle, le pourcentage étant calculé par rapport à la longueur totale du couvercle.

6. Procédé selon l'une quelconque des revendications 1 à 5, dans lequel les moyens permettant de maintenir le couvercle et le corps en position rigoureusement coaxiale sont constitués par au moins trois saillies, de préférence selon un agencement symétrique, de préférence des saillies adjacentes équidistantes les unes des autres.

7. Procédé selon la revendication 6, dans lequel lesdits moyens sont constitués de 4, 5, 6, 7, 8, 9 ou 10 saillies, de préférence 6, 7, 8, 9 ou 10, en particulier 6 ou 8 saillies, de préférence selon une disposition symétrique, toutes les saillies ayant de préférence la même forme, et les saillies de préférence adjacentes étant toutes équidistantes les unes des autres.

8. Procédé selon l'une quelconque des revendications 1 à 5, dans lequel lesdits moyens sont une nervure annulaire, de préférence interrompue.

9. Procédé selon l'une quelconque des revendications 1 à 8, dans lequel il y a deux moyens permettant de maintenir le couvercle et le corps en position rigoureusement coaxiale.

10. Procédé selon l'une quelconque des revendications 1 à 9, dans lequel les moyens permettant de maintenir le couvercle et le corps en position rigoureusement coaxiale sont situés à une distance comprise entre 50 et 95%, de préférence entre 50 et 85%, en particulier entre 55 et 80% et tout spécialement entre 65 et 75% de la longueur totale du couvercle, les pourcentages étant calculés à partir du sommet de l'extrémité fermée du couvercle vers son extrémité ouverte.

11. Procédé selon la revendication 10, dans lequel il y a deux saillies situées entre 65 et 75% de la longueur totale du couvercle, les pourcentages étant calculés à partir du sommet de l'extrémité fermée du couvercle, vers son extrémité ouverte.

12. Procédé selon l'une quelconque des revendications 1 à 11, dans lequel la partie de corps présente une surface extérieure lisse.

13. Procédé selon l'une quelconque des revendications 1 à 12, dans lequel la partie de corps présente sur sa surface extérieure des rainures annulaires ou un agencement de rainures en correspondance de forme avec les moyens de nervure de la surface intérieure du couvercle, de façon à assurer un verrouillage complet, pratiquement sans distorsion, entre le couvercle et le corps.

14. Procédé selon l'une quelconque des revendications 1 à 13, dans lequel les nervures annulaires et les rainures sont interrompues de façon que les espaces entre les segments de nervure jouent le rôle d'évents pour permettre à l'air de s'échapper de l'intérieur de la gélule quand elle est assemblée.

**15.** Procédé selon l'une quelconque des revendications 1 à 14, dans lequel la nervure annulaire du couvercle est un étranglement du diamètre de ce dernier.

**16.** Procédé selon l'une quelconque des revendications 1 à 15, dans lequel la nervure annulaire comporte deux pentes et, entre elles, une éventuelle surface plane.

**17.** Procédé selon l'une quelconque des revendications 1 à 16, dans lequel les saillies ont une base ovale, circulaire ou rectangulaire et présentent en section transversale deux pentes et, entre ces dernières, une éventuelle surface plane.

**18.** Procédé selon l'une quelconque des revendications 1 à 17, dans lequel le corps présente un diamètre réduit de sa paroi extérieure dans la zone de son extrémité ouverte, par comparaison au diamètre du reste de la paroi extérieure.

**19.** Procédé selon la revendication 18, dans lequel l'étranglement du corps est en correspondance de forme avec l'extrémité fermée du couvercle ou avec un étranglement de ce dernier, pour assurer un joint d'étanchéité mécanique quand la gélule est fermée.

**20.** Procédé selon l'une quelconque des revendications 1 à 17, dans lequel la longueur du corps est ajustée de façon à correspondre à l'extrémité fermée du couvercle ou d'un étranglement du couvercle de façon à assurer un joint d'étanchéité mécanique quand la gélule est fermée.

**21.** Procédé selon l'une quelconque des revendications 1 à 20, dans lequel la paroi latérale du corps cylindrique est entièrement enfermée dans la paroi latérale intérieure du couvercle quand la gélule est assemblée.

**22.** Procédé selon l'une quelconque des revendications 1 à 21, qui consiste à étancher des gélules de gélatine obtenues par moulage au trempé.

**23.** Procédé selon l'une quelconque des revendications 1 à 21, qui consiste à étancher des gélules obtenues à partir de gélatine ou de dérivés de l'amidon ou de leurs mélanges, ou d'amidon natif, par moulage par injection.

**24.** Procédé selon l'une quelconque des revendications 1 à 22, dans lequel la paroi de la partie de couvercle et/ou de corps constitue une mousse.

**25.** Procédé selon l'une quelconque des revendications 1 à 24, consistant à étancher des gélules contenant des produits alimentaires, des produits pharmaceutiques, des produits chimiques, des colorants, des épices, des combinaisons fertilisantes, des semences, des cosmétiques et des produits agricoles sous forme de poudre, de pâte ou de liquide.

**26.** Procédé selon l'une quelconque des revendications 1 à 25, pour contenir un liquide.

**27.** Procédé selon la revendication 26, dans lequel la mise en contact du bord de la partie de couvercle de la gélule avec le liquide d'étanchéité s'effectue par immersion dans le fluide, pulvérisation, mise en contact avec un matériau solide imprégné ou mouillé du fluide d'étanchéité, de préférence au trempé ou par pulvérisation, en particulier par pulvérisation.

**28.** Procédé selon l'une quelconque des revendications 26 et 27, dans lequel le fluide d'étanchéité en excès est enlevé par agitation, vibration, soufflage à l'air ou une combinaison de ces techniques, de préférence par soufflage à l'air à température ambiante, en particulier dans un lit fluidisé.

**29.** Procédé selon l'une quelconque des revendications 27 et 28, dans lequel l'étanchéité est mise en oeuvre à température ambiante.

**30.** Procédé selon l'une quelconque des revendications 27 et 28, dans lequel l'étanchéité est mise en oeuvre à une température supérieure à 30°C, de préférence de 30 à 80°C, en particulier de 35 à 70°C et tout spécialement de 40 à 60°C.

**31.** Procédé selon l'une quelconque des revendications 27 à 29, dans lequel l'étanchéité est mise en oeuvre dans un lit fluidisé à l'aide d'air chauffé.

**32.** Procédé selon l'une quelconque des revendications 27 et 28, dans lequel l'étanchéité est mise en oeuvre avec une énergie électromagnétique, de préférence un rayonnement infrarouge.

**33.** Procédé selon l'une quelconque des revendications 27 à 32, dans lequel le fluide d'étanchéité est un liquide organique aqueux soluble dans l'eau et contenant de l'eau.

**34.** Procédé selon l'une quelconque des revendications 27 à 33, dans lequel le fluide d'étanchéité est un mono-alcool aliphatique comportant de 1 à 4 atomes de carbone pouvant être substitués par un groupe alkoxy ayant 1 ou 2 atomes de carbone, ou leurs mélanges contenant 2 à 90% d'eau, de préférence de 5 à 70% d'eau.

**35.** Procédé selon l'une quelconque des revendications 27 à 34, dans lequel le liquide d'étanchéité est un mélange d'éthanol et d'eau.

**36.** Procédé selon la revendication 35, dans lequel le fluide d'étanchéité contient plus de 40% d'éthanol, de préférence 40 à 95% et tout spécialement 45 à 83%.

**37.** Gélules rendues étanches selon l'une quelconque des revendications 1 à 36.

**38.** Une gélule en gélatine dure comportant des parties de couvercle et de corps coaxiales, cylindriques, pouvant être emboîtées de façon télescopique, chacune d'elles comportant une paroi latérale, une extrémité ouverte et une extrémité fermée, le couvercle et le corps étant susceptibles d'être assemblés l'un à l'autre, la gélule étant une gélule dans laquelle la partie de couvercle comporte sur sa paroi intérieure une nervure annulaire ou un agencement jouant le rôle d'une nervure annulaire, caractérisée en ce que la ou les nervure(s), ou le ou les agencement(s) est(sont) situé(s) à la partie supérieure du couvercle, au voisinage de son extrémité fermée, dans les 50% supérieurs de la longueur du couvercle, calculée par rapport à la longueur totale de ce dernier, et en ce que, à une certaine distance de ladite nervure ou dudit agencement, vers l'extrémité ouverture du couvercle, il est prévu des moyens comprenant au moins trois saillies, à une distance suffisante pour maintenir le couvercle et le corps en position rigoureusement coaxiale, lesdits moyens étant situés à une distance comprise entre 50 et 90% de la longueur totale du couvercle, ce pourcentage étant calculé à partir du sommet de l'extrémité fermée du couvercle, vers son extrémité ouverte.

**39.** Une gélule en gélatine dure selon la revendication 38, dans laquelle à distance, vers l'extrémité ouverte de la partie de couvercle, sont disposés des moyens sous la forme de 4, 5, 6, 7, 8, 9 ou 10 saillies.

**Ansprüche**

**1.** Verfahren zum Versiegeln harter Gelatinekapseln mit koaxialen Kappen- und Körperteilen, welche bei teleskopischem Zusammenfügen überlappen, durch Kontaktieren der Kante des Kappenteils der Kapsel mit der Versiegelungsflüssigkeit, sodaß sie innerhalb dieses überlappenden Abschnitts gleichmäßig verteilt wird, (ii) Entfernen des Überschusses der Versiegelungsflüssigkeit von der freiliegenden Außenfläche, sodaß nur der überlappende Abschnitt benetzt bleibt, und (iii) Bewirken des Versiegelns der Kapsel innerhalb des überlappenden Abschnitts der Kapselkörper- und -kappenteile durch Verweilenlassen der Kapsel in Umgebungstemperatur oder durch Aufbringen von Hitze auf den überlappenden Abschnitt, dadurch gekennzeichnet, daß eine Kapsel verwendet wird, deren Kappenteil auf seiner Innenwandfläche eine ringförmige Rippe oder eine wie eine ringförmige Rippe wirkende Anordnung aufweist, welche Anordnung vorzugsweise eine Anordnung von Rippensegmenten und/oder Vorwölbungen ist, und mit Abstand von der Rippe oder Anordnung, in Richtung des offenen Endes des Kappenteils, ein Mittel in einem Abstand angeordnet ist, welches ausreicht, Kappe und Körper in einer genau koaxialen Position zu halten.

**2.** Verfahren nach Anspruch 1, worin die ringförmige Rippe des Kappenteils eine Rippe in Umfangsrichtung ist, welche gegebenenfalls unterbrochen ist.

**3.** Verfahren nach Anspruch 1, worin die ringförmige Rippe des Kappenteils eine Anordnung von in Umfangsrichtung angeordneten Rippensegmenten oder Vorwölbungen ist.

**4.** Verfahren nach irgendeinem der Ansprüche 1 bis 3, worin der Kappenteil eine zusätzliche ringförmige Rippe oder eine zusätzliche Anordnung von Rippensegmenten und/ oder Vorwölbungen aufweist.

**5.** Verfahren nach irgendeinem der Ansprüche 1 bis 4, worin sich die Rippe(n) im oberen Teil der Kappe, nahe ihrem geschlossenen Ende, innerhalb der oberen 50 % der Kappenlänge, auf die Gesamtlänge der Kappe bezogen berechnet, befindet(n).

**6.** Verfahren nach irgendeinem der Ansprüche 1 bis 5, worin das die Kappe und den Körper in einer genau koaxialen Position haltende Mittel durch mindestens drei Vorwölbungen, vorzugsweise in symmetrischer Anordnung,repräsentiert ist, wobei vorzugsweise benachbarte Vorwölbungen alle den gleichen Abstand voneinander haben.

7. Verfahren nach Anspruch 6, worin als diese Mittel 4, 5, 6, 7, 8, 9 oder 10 Vorwölbungen, vorzugsweise 6, 7, 8, 9 oder 10, vorzugsweise 6 oder 8 Vorwölbungen, vorzugsweise in einer symmetrischen Anordnung, angeordnet sind, wobei vorzugsweise alle Vorwölbungen die gleiche Form haben, und vorzugsweise benachbarte Vorwölbungen alle den gleichen Abstand voneinander aufweisen.

8. Verfahren nach irgendeinem der Ansprüche 1 bis 5, worin das Mittel eine ringförmige Rippe ist, welche vorzugsweise unterbrochen ist.

9. Verfahren nach irgendeinem der Ansprüche 1 bis 8, worin zwei Mittel vorgesehen sind, um Kappe und Körper in einer genau koaxialen Position zu halten.

10. Verfahren nach irgendeinem der Ansprüche 1 bis 9, worin das zum Halten von Kappe und Körper in genau koaxialer Position dienende Mittel innerhalb von 50 bis 95%, vorzugsweise innerhalb von 50 bis 85%, vorzugsweise innerhalb von 55 bis 80%, und vorzugsweise innerhalb von 65 bis 75 % der Kappengesamtlänge, von der Spitze des geschlossenen Endes der Kappe in Richtung ihres offenen Endes berechnet, liegt.

11. Verfahren nach Anspruch 10, worin zwei Vorwölbungen innerhalb eines Bereichs von 65 bis 75 % der Kappengesamtlänge, von der Spitze des geschlossenen Endes der Kappe in Richtung ihres offenen Endes berechnet, vorgesehen sind.

12. Verfahren nach irgendeinem der Ansprüche 1 bis 11, worin der Körperteil eine glatte Außenfläche aufweist.

13. Verfahren nach irgendeinem der Ansprüche 1 bis 12, worin der Körperteil auf seiner Außenfläche ringförmige Nuten oder eine Anordnung von Nuten, welche mit den Rippenmitteln der Innenfläche der Kappe zusammenpassen, um eine im wesentlichen verzerrungsfreie vollkommene Verriegelung zwischen der Kappe und dem Körper vorzusehen, aufweist.

14. Verfahren nach irgendeinem der Ansprüche 1 bis 13, worin die ringförmigen Rippen und Nuten so unterbrochen sind, daß die Zwischenräume zwischen den Rippensegmenten als Entlüftungsöffnungen dienen,um beim Zusammenfügen Luft aus dem Innern der Kapsel entweichen zu lassen.

15. Verfahren nach irgendeinem der Ansprüche 1 bis 14, worin die ringförmige Rippe der Kappe eine Einschnürung des Kappendurchmessers ist.

16. Verfahren nach irgendeinem der Ansprüche 1 bis 15, worin die ringförmige Rippe zwei Schrägen und gegebenenfalls eine ebene Fläche dazwischen aufweist.

17. Verfahren nach irgendeinem der Ansprüche 1 bis 16, worin die Vorwölbungen eine ovale, runde oder rechteckige Basis haben und im Querschnitt zwei Schrägen und gegebenenfalls eine ebene Fläche dazwischen zeigen.

18. Verfahren nach irgendeinem der Ansprüche 1 bis 17, worin der Körper im Bereich seines offenen Endes einen verkürzten Durchmesser der Außenwand, verglichen mit dem Durchmesser der restlichen Außenwand, aufweist.

19. Verfahren nach Anspruch 18, worin die Einschnürung des Körpers mit dem geschlossenen Ende der Kappe oder einer Einschnürung der Kappe zusammenpaßt, um eine dichte mechanische Versiegelung zu ergeben, wenn die Kapsel geschlossen ist.

20. Verfahren nach irgendeinem der Ansprüche 1 bis 17, worin die Länge des Körpers so festgesetzt ist, daß sie mit dem geschlossenen Ende der Kappe oder einer Einschnürung der Kappe zusammenpaßt, um eine dichte mechanische Versiegelung zu ergeben, wenn die Kapsel geschlossen ist.

21. Verfahren nach irgendeinem der Ansprüche 1 bis 20, worin die zylindrische Körperseitenwand vollkommen innerhalb der inneren Kapselseitenwand eingeschlossen ist, wenn die Kapsel zusammengefügt ist.

22. Verfahren nach irgendeinem der Ansprüche 1 bis 21 zur Versiegelung von Gelatinekapseln, welche mittels des Tauchgußverfahrens hergestellt werden.

23. Verfahren nach irgendeinem der Ansprüche 1 bis 21 zur Versiegelung von aus Gelatine oder Stärkederivaten oder Mischungen derselben oder aus natürlicher Stärke durch Spritzguß hergestellten Kapseln.

24. Verfahren nach irgendeinem der Ansprüche 1 bis 22, worin die Wand des Kappen- und/oder des Körperteils einen Schaum repräsentiert.

25. Verfahren nach irgendeinem der Ansprüche 1 bis 24 zur Versiegelung von Kapseln, welche Lebensmittel, Pharmazeutika, Chemikalien, Farbstoffe, Gewürze, Düngemittelkombinationen, Samen, Kosmetika und landwirtschaftliche Produkte als Pulver, Paste oder Flüssigkeit enthalten.

26. Verfahren nach irgendeinem der Ansprüche 1 bis 25 umfassend eine Flüssigkeit.

27. Verfahren nach Anspruch 26, worin das Kontaktieren der Kante des Kappenteils der Kapsel mit der Versiegelungsflüssigkeit durch Eintauchen in das Fluid, Sprühen, Kontaktieren eines mit dem Versiegelungsfluid imprägnierten oder benetzten festen Materials, vorzugsweise durch Eintauchen oder Sprühen, insbesondere durch Sprühen, erfolgt.

28. Verfahren nach irgendeinem der Ansprüche 26 und 27, worin das überschüssige Versiegelungsfluid durch Schütteln, Schwingen, Abblasen mit Luft oder einer Kombination davon, vorzugsweise durch Abblasen mit Luft bei Umgebungstemperatur, vorzugsweise in einem Wirbelbett, entfernt wird,

29. Verfahren nach irgendeinem der Ansprüche 27 und 28, worin das Versiegeln bei Umgebungstemperatur durchgeführt wird.

30. Verfahren nach irgendeinem der Ansprüche 27 und 28, worin das Versiegeln bei einer Temperatur, welche höher als 30°C ist, vorzugsweise bei 30 bis 80°C, vorzugsweise bei 35 bis 70°C, und vorzugsweise bei 40 bis 60°C durchgeführt wird.

31. Verfahren nach irgendeinem der Ansprüche 27 bis 29, worin das Versiegeln in einem Wirbelbett mit erhitzter Luft durchgeführt wird.

32. Verfahren nach irgendeinem der Ansprüche 27 bis 28, worin das Versiegeln mit elektromagnetischer Energie, vorzugsweise mit Infrarotwellen durchgeführt wird.

33. Verfahren nach irgendeinem der Ansprüche 27 bis 32, worin das Versiegelungsfluid eine wasserlösliche organische Flüssigkeit, welche Wasser enthält, ist.

34. Verfahren nach irgendeinem der Ansprüche 27 bis 33, worin das Versiegelungsfluid ein aliphatischer einwertiger Alkohol mit einem bis vier Kohlenstoffatomen, welche durch eine Alkoxygruppe mit einem oder zwei Kohlenstoffatomen oder einer Mischung davon substituiert sein können, welcher 2 bis 90% Wasser, vorzugsweise 5 bis 70 % Wasser enthält, ist.

35. Verfahren nach irgendeinem der Ansprüche 27 bis 34, worin das Versiegelungsfluid eine Ethanol/Wasser-Mischung ist.

36. Verfahren nach Anspruch 35, worin das Versiegelungsfluid mehr als 40 % Ethanol, vorzugsweise 40 bis 95%, insbesondere 45 bis 83%, enthält.

37. Kapseln, welche nach irgendeinem der Ansprüche 1 bis 36 versiegelt sind.

38. Harte Gelatinekapsel, welche Kapsel zylindrische teleskopisch zusammenfügbare koaxiale Kappen- und Körperteile aufweist, welche jeweils eine Seitenwand, ein offenes Ende und ein geschlossenes Ende haben, wobei die Kappe und der Körper zur wechselseitigen Verbindung geformt sind, worin die Kapsel so gestaltet ist, daß der Kappenteil auf seiner Innenwandfläche eine ringförmige Rippe oder eine wie eine ringförmige Rippe wirkende Anordnung aufweist, dadurch gekennzeichnet, daß die Rippe(n) oder Anordnung(en) am oberen Teil der Kappe, nahe ihrem geschlossenen Ende, innerhalb der oberen 50 % der Kappenlänge, auf die Gesamtlänge der Kappe bezogen berechnet, liegt(gen), und daß mit Abstand von der Rippe oder der Anordnung,in Richtung des offenen Endes des Kappenteils, ein zumindest drei in Abständen angeordnete Vorwölbungen umfassendes Mittel, welches ausreicht, die Kappe und den Körper in einer genau koaxialen Position zu halten, angeordnet ist, welches Mittel innerhalb von 50 bis 95% der Kappengesamtlänge, berechnet von der Spitze des geschlossenen Endes der Kappe, in Richtung ihres offenen Endes, liegt.

39. Harte Gelatinekapsel nach Anspruch 38, worin in Richtung des offenen Endes des Kappenteils ein Mittel in Form von 4, 5, 6, 7, 8, 9 oder 10 Vorwölbungen in Abständen angeordnet ist.

Fig. 1a

Fig. 1b

Fig. 1c

Fig. A

Fig.2

Fig.3

Fig.4

_Fig. 5_

_Fig. 6_

_Fig. 7_

_Fig. 8_

Fig. 9

Fig. 10

Fig. 11

Fig. 12